(19) Europäisches Patentamt
European Patent Office
Office européen des brevets

(11) EP 0 597 304 B1

(12) **EUROPÄISCHE PATENTSCHRIFT**

(45) Veröffentlichungstag und Bekanntmachung des Hinweises auf die Patenterteilung:
**10.01.2001 Patentblatt 2001/02**

(51) Int Cl.[7]: **A01N 43/52**, A61K 31/415

(21) Anmeldenummer: **93117243.1**

(22) Anmeldetag: **25.10.1993**

(54) **Verwendung von substituierten Benzimidazolen zur Bekämpfung parasitärer Protozoen**

Use of substituted benzimidazoles for combating parasitic protozoas

Utilisation de benzimidazoles substitués pour combattre les protozoaires parasites

(84) Benannte Vertragsstaaten:
**BE CH DE DK ES FR GB GR IT LI NL SE**

(30) Priorität: **06.11.1992 DE 4237617**

(43) Veröffentlichungstag der Anmeldung:
**18.05.1994 Patentblatt 1994/20**

(73) Patentinhaber: **BAYER AG**
**51368 Leverkusen (DE)**

(72) Erfinder:
- **Lunkenheimer, Winfried, Dr.**
  **D-42115 Wuppertal (DE)**
- **Baasner, Bernd, Dr.**
  **D-51467 Bergisch Gladbach (DE)**
- **Lieb, Folker, Dr.**
  **D-51375 Leverkusen (DE)**
- **Haberkorn, Axel, Prof. Dr.**
  **D-42119 Wuppertal (DE)**

(56) Entgegenhaltungen:
**DE-A- 1 545 816**
**DE-A- 1 670 786**
**DE-A- 2 047 369**
**DE-A- 2 736 448**
**FR-A- 1 439 128**
**FR-A- 1 476 531**
**GB-A- 1 022 659**
**GB-A- 1 213 796**
**GB-A- 1 505 846**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft die Verwendung von substituierten Benzimidazolen als Mittel zur Bekämpfung parasitärer Protozoen, insbesondere Coccidien.

Substituierte Benzimidazole, Mischungen enthaltend dieselben und ihre Verwendung als Insektizide, Fungizide, Parasitizide und Herbizide sind bereits bekannt geworden (EP-OS 87 375, 152 360, US-P 3 472 865, 3 576 818, US-P 3 418 318, EP-OS 260 744, 266 984, 181 826, 239 508, GB-OS 1 505 846, DE-OS 1 545 816, GB-OS 1 213 796).

Polyhalogenierte Benzimidazole, Mittel enthaltend dieselben und ihre Wirkung als Anthelmintika, Coccidiostatika und Pestizide sind ebenfalls bekannt geworden (DE-OS 2 047 369, GB 1 022 659, DE-OS 27 36 448, FR-OS 1 476 531, 1 439 128). Doch befriedigt ihre Wirkung nicht in jedem Fall.

Gegenstand der vorliegenden Erfindung ist die Verwendung von substituierten Benzimidazolen der Formel (I)

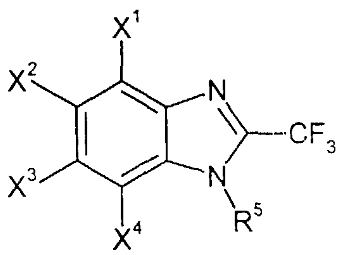

(I),

in welcher

$X^1$, $X^2$, $X^3$ und $X^4$ unabhängig voneinander jeweils für Wasserstoff, Fluor, Chlor, Brom, Iod, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschieden Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 5 Kohlenstoffatomen stehen,

wobei jedoch mindestens einer der Substituenten $X^1$, $X^2$, $X^3$ und $X^4$ verschieden von Wasserstoff und Halogen ist,

$R^5$ für $C_{14}$-Alkyl steht, das ein- oder mehrfach, gleich oder verschieden substituiert ist durch OH, CN, $NH_2$, $C_{1-6}$-Alkoxy, 1 bis 5 Halogen-$C_{1-6}$-Alkoxy, $C_{1-6}$-Alkylthio, 1 bis 5 Halogen-$C_{1-6}$-alkylthio, $C_{2-6}$-Alkenoxy, $C_{3-6}$-Alkinoxy, Amino oder Mono-($C_{1-6}$-alkyl)-amino, die acyliert sein können,

wobei die Acylreste der aufgeführten acylierten Reste folgende Bedeutung haben: $C_{1-6}$-Alkoxycarbonyl, $C_{1-6}$-Alkylcarbonyl,
zur Herstellung von Mitteln zur Bekämpfung parasitärer Protozoen und Coccidien.

Die substituierten Benzimidazole der Formel (I) sind zum Teil bekannt und können nach an sich bekannten Verfahren hergestellt werden.

Zum Teil sind die substituierten Benzimidazole der Formel (I) neu und Gegenstand der gleichzeitig eingereichten Patentanmeldungen EP-A 0 667 861, 0 667 863 und 0 667 862 der Anmelderin.

Besonders bevorzugt haben in Verbindungen der Formel (I) die Substituenten folgende Bedeutungen:

$X_1$ und $X_4$ stehen für gleiche oder verschiedene Reste aus der Gruppe H, $C_{1-4}$-Alkyl, das durch Fluor oder Chlor perhalogeniert ist, Halogen, wobei

einer der Reste $X^2$ oder $X^3$ für halogen-substituierte Reste der Gruppe $C_{1-4}$-Alkyl, Alkylthio steht und der andere Rest $X^2$ oder $X^3$ für Wasserstoff oder Halogen steht;
die Reste $X^2$ und $X^3$ können auch gemeinsam mit den angrenzenden C-Atomen für einen Dioxolanyl- oder Dioxanylring stehen, die durch Halogen, Trifluormethyl oder Trifluorethyl substituiert sein können;

$R^5$ steht für Methyl, das substituiert ist durch OH, CN, $C_{1-4}$-Halogenalkoxy, Amino oder Mono-($C_{1-4}$-alkyl)amino, die

acyliert sein können durch $C_{1-4}$-Alkoxycarbonyl.

Ganz besonders bevorzugt sind Verbindungen der Formel (I), in welcher die Reste folgende Bedeutungen haben:

$X^1$ und $X^4$ stehen jeweils unabhängig voneinander für gleiche oder verschiedene Reste aus der Gruppe Wasserstoff, Chlor, Brom, Trifluormethyl;

einer der Reste $X^2$ oder $X^3$ steht für Trifluormethyl,
der andere Rest $X^2$ oder $X^3$ steht für Wasserstoff oder Halogen,

$R^5$ hat die oben angegebenen Bedeutungen.

Die substituierten Benzimidazole der allgemeinen Formel (I) werden erhalten wenn man 1H-Benzimidazole der Formel (II)

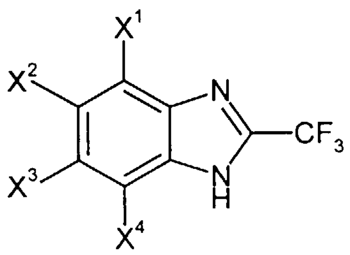

(II)

in welcher

$X^1$, $X^2$, $X^3$ und $X^4$ die oben angegebene Bedeutung haben,

mit Verbindungen der Formel (III)

$$A\text{-}R^5 \qquad \text{(III)}$$

in welcher

A für eine geeignete Abgangsgruppe steht, und

$R^5$ die oben angegebene Bedeutung hat

gegebenenfalls in Gegenwart eines Verdünnungsmittels und gegebenenfalls in Gegenwart eines Reaktionshilfsmittels umsetzt.

Die zur Durchführung des Verfahrens weiterhin als Ausgangsprodukte erforderlichen Verbindungen sind durch die Formel (III) allgemein definiert. In dieser Formel (III) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

A steht vorzugsweise für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

A steht außerdem auch für eine Alkohol-, Alkanoyloxy- oder Alkoxygruppe, wie beispielsweise eine Hydroxy-, Acetoxy- oder Methoxygruppe.

Die Verbindungen der Formel (III) sind bekannt oder erhältlich in Analogie zu bekannten Verfahren (vgl. z.B. DE 20 40 175; DE 21 19 518; Synthesis 1973, 703).

Als Verdünnungsmittel zur Durchführung des Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Basen wie Pyridin oder organische Säuren, wie Ameisensäure oder Essigsäure.

Das Verfahren wird vorzugsweise in Gegenwart eines geeigneten Reaktionshilfsmittels durchgeführt. Als solche kommen alle üblichen anorganischen oder organischen Basen in Frage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Lithium-diethylamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, Lithium-organische Verbindungen, wie n-Butyllithium sowie tertiäre Amine, wie Trimethylamin, Triethylamin, Tributylamin, Di-isopropyl-ethylamin, Tetramethylguanidin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

In den Fällen, wo A in Formel (III) für eine Alkohol-, Alkanoyloxy- oder Alkoxygruppe steht, kommen als Reaktionshilfsmittel auch organische oder anorganische Säuren, wie beispielsweise Schwefelsäure, Salzsäure, p-Toluolsulfonsäure, Perfluorbutansulfonsäure oder stark saure Ionenaustauscher in Frage.

Das Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid. Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-$C_{12}$/$C_{14}$-alkylbenzylammoniumchlorid, Dimethyl-$C_{12}$/$C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)ethyl]-amin.

Die Reaktionstemperaturen können bei der Durchführung des Verfahrens in einem größeren Bereich variiert werden. Im allgemeinen arbeitet man bei Temperaturen zwischen -70°C und +200°C, vorzugsweise bei Temperaturen zwischen 0°C und 130°C.

Das Verfahren wird üblicherweise unter Normaldruck durchgeführt. Es ist jedoch auch möglich unter erhöhtem oder vermindertem Druck zu arbeiten.

Zur Durchführung des Verfahrens setzt man pro Mol an 1H-Benzimidazol der Formel (II) im allgemeinen 1,0 bis 5,0 Mol, vorzugsweise 1,0 bis 2,5 Mol an Verbindung der Formel (III) und gegebenenfalls 0,01 bis 5,0 Mol, vorzugsweise 1,0 bis 3,0 Mol an Reaktionshilfsmittel ein.

In einer besonderen Durchführungsform ist es auch möglich, die 1H-Benzimidazole der Formel (II) zunächst in einem vorgelagerten Reaktionsschritt mit Hilfe üblicher Silylierungsverfahren beispielsweise mit Hexamethyldisilazan oder Trimethylsilylchlorid, gegebenenfalls in Gegenwart eines geeigneten Katalysators, wie beispielsweise Schwefelsäure, Trifluoressigsäure, Ammoniumsulfat, Imidazol oder Saccharin bei Temperaturen zwischen -20°C und +50°C zu silylieren und die so erhältlichen 1-Trimethylsilylbenzimidazole in einer anschließenden zweiten Stufe mit Alkylierungsmitteln der Formel (II) gemäß dem erfindungsgemäßen Verfahren umzusetzen. In diesem Fall ist es von Vorteil als Katalysator zur Alkylierungsreaktion Zinntetrachlorid zuzusetzen (vgl. z.B. Chem. Heterocycl. Com. USSR 24, 514 (1988)).

Die Reaktionsdurchführung, Aufarbeitung und Isolierung der Reaktionsprodukte erfolgt nach bekannten Verfahren (vgl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Im einzelnen seien die folgenden substituierten Benzimidazole der allgemeinen Formel (I)

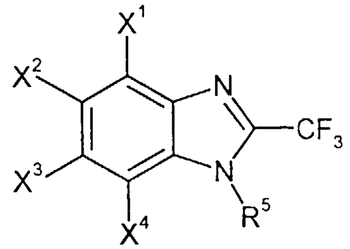

(I),

mit

$R^5$ =

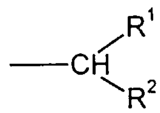

genannt:

| $X^1$ | $X^2$ | $X^3$ | $X^4$ | $R^1$ | $R^2$ |
|---|---|---|---|---|---|
| Br | H | Cl | H | H | 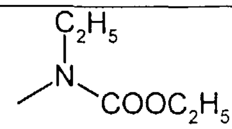 |
| Br | H | Br | H | H | 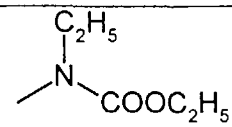 |
| H | Cl | Cl | H | H | 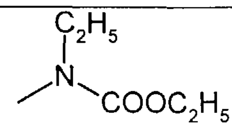 |
| Cl | H | Cl | H | H | 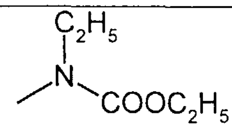 |
| Cl | H | Br | H | H | 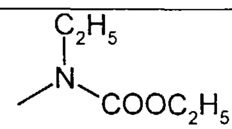 |
| Br | H | Cl | H | H | $-O-C_2H_5$ |
| Br | H | Br | H | H | $-O-C_2H_5$ |
| H | Cl | Cl | H | H | $-O-C_2H_5$ |
| Cl | H | Cl | H | H | $-O-C_2H_5$ |
| Cl | H | Br | H | H | $-O-C_2H_5$ |

Die Wirkstoffe eignen sich bei günstiger Warmblütertoxizität zur Bekämpfung von parasitischen Protozoen die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthomoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E, chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. maleagrimitis, E. mitis, E. necatrix, E. ninahohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovithominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z. B. Babesia argentina, B. ovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japenica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channell catfish. Besonders geeignet sind die erfindungsgemäßen Mittel zur Behandlung von Fischbrut, z.B. Karpfen von 2 bis 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgussformulierungen, Gele;

Emulsionen und Suspensionen zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltiger Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt. wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glykole, Polyethylenglykole, Polypropylenglykole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglykol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Naltrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm bis 20 Gew.-%, bevorzugt von 0,1 bis 10 Gew.-%.

Zubereitungen die vor Anwendung verdünnt werden, enthalten den Wirkstoff in Konzentrationen von 0,5 bis 90 Gew.-%, bevorzugt von 1 bis 50 Gew.-%.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten, essbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem essbaren organischen oder anorganischen Träger enthält, mit üblichen Futtermitteln. Essbare Träger sind z.B. Maismehl oder Mais- und Sojabohnenmehl oder Mineralsalze, die vorzugsweise eine geringe Menge eines essbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten essbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen

abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muss. Bei der Verabreichung größerer Mengen kann es zweckmäßig sein, diese im Verlauf des Tages in mehrere Einzeldarreichungen zu unterteilen.

Zu den Parasiten bei Fischen gehören aus dem Unterreich der protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Fleistophora spp..

Die Behandlung der Fische erfolgt entweder oral, z.B. über das Futter oder durch Kurzzeitbehandlung, "medizinisches Bad", in das die Fische eingesetzt und in dem sie eine Zeitlang (Minuten bis mehrere Stunden) z.B. beim Umsetzen von einem Zuchtbecken zum anderen gehalten werden.

Es kann aber auch eine vorübergehende oder dauernde Behandlung des Lebensraums der Fische (z.B. ganzer Teichanlagen, Aquarien, Tanks oder Becken), in denen die Fische gehalten werden, erfolgen.

Der Wirkstoff wird in Zubereitungen verabreicht, die den Anwendungen angepasst sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7 bis 10, vorzugsweise aber einen pH-Wert von 8 bis 10 haben.

Die Konzentration des Wirkstoffes kann im Bereich von 0,5 bis 50 % liegen, vorzugsweise aber in einem Bereich von 1 bis 25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly (oxyethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N',N'-Tetrakis-(2-hydroxypropyl)-ethylendiamin oder Polyether-Tetrol auf der Basis Ethylendiamin (MG. 480 bis 420), anorganische Basen, wie Ammoniak oder Natriumcarbonat, gegebenenfalls unter Zugabe von Wasser.

Die Zubereitungen können auch 0,1 bis 20 Gew.-%, vorzugsweise 0,1 bis 10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2 bis 50 mg Wirkstoff pro Liter Wasser, bevorzugt 5 bis 10 mg pro Liter, bei einer Behandlungsdauer von 3 bis 4 Stunden. Bei der Behandlung von jungen Karpfen wird z.B. mit einer Konzentration von 5 bis 10 mg/l und einer Behandlungsdauer von ca. 1 bis 4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1 bis 5 mg Wirkstoff pro Liter Wasser verwendet werden.

| | | |
|---|---|---|
| a) | Wirkstoff der Formel (I)<br>Sojabohnen-Protein | 1-10 Gew.-Teile<br>49-90 Gew.-Teile |

(fortgesetzt)

| | | |
|---|---|---|
| b) | Wirkstoff der Formel (I)<br>Benzylalkohol<br>Hydroxypropylmethylcellulose<br>Wasser | 0,5-10 Gew.-Teile<br>0,08-1,4 Gew.-Teile<br>0-3,5 Gew.-Teile<br>Rest ad 100 |

Zubereitungen zur Anwendung bei "medizinischen Bädern" und zur Teichbehandlung sind z.B. wie folgt zusammengesetzt und hergestellt.

| | | |
|---|---|---|
| c) | 2,5 g | Wirkstoff der Formel (I) werden in 100 ml Triethanolamin unter Erwärmen belöst. |
| d) | 2,5 g Wirkstoff der Formel (I)<br>12,5 g Milchsäure werden in 100 ml Triethanolamin unter Erwärmen und Rühren gelöst. | |
| e) | 10,0 g Wirkstoff der Formel (I) wird in 100 ml Monoethanolamin gelöst. | |
| f) | Wirkstoff der Formel (I)<br>Propylenglykol<br>Natriumcarbonat<br>Wasser | 5,0 g<br>50,0 g<br>5,0 g<br>ad 100 ml |
| g) | Wirkstoff der Formel (I)<br>Monoethanolamin<br>N-Methylpyrrolidon | 5,0 g<br>10 g<br>ad 100 ml |
| h) | Wirkstoff der Formel (I)<br>Natriumcarbonat<br>Polyethylenglykol 200 | 2,5 g<br>5,0 g<br>ad 100 ml |

Der Wirkstoff wird unter Erwärmen im Polyethylenglykol gelöst und Natriumcarbonat darin suspendiert.

## Beispiel A

Coccidiose bei Hühnern

9 bis 11 Tage alte Küken wurden mit 40 000 sporulierten Oozysten von stark virulenten Stämmen von Eimeria acervulina, E. maxima und E. tenella, den Krankheitserregern der intestinalen Coccidiose infiziert.
3 Tage vor der Infektion und 8 Tage nach der Infektion (Ende des Versuchs) wurde Wirkstoff in der angegebenen Konzentration im Futter der Tiere eingemischt verabreicht.
Die Zahl der Oozysten im Kot wurde mit Hilfe der McMaster-Kammer bestimmt (siehe Engelbrecht und Mitarbeiter "Parasitologische Arbeitsmethoden in Medizin und Vererinärmedizin", S. 172, Akademie-Verlag, Berlin (1965)).
In der folgenden Tabelle werden Wirkstoffdosen und Oozystenausscheidung in % für die einzelnen Erreger angegeben. Dabei bedeutet 100 % keine Wirkung und 0 % volle Wirkung d.h. keine Oozystenausscheidung.

Tabelle 1

| Coccidiose bei Hühern | | | | |
|---|---|---|---|---|
| Bsp. Nr. | Dosis ppm. | E.acervulina Oocystenausscheidung in % im Vergleich zur unbehandelten infizierten Kontrolle | E.maxima Oocytenausscheidung in % im Vergleich zur nicht infizierten unbehandelten Kontrolle | E.tenella |
| unbehandelte infizierte Kontrolle | 0 | 100 | 100 | 100 |
| | 50 | 0 | 0 | 0 |

Beispiele für die Herstellung der Verbindungen I:

**Beispiel I-1**

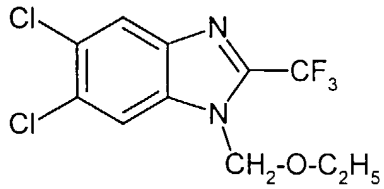

In eine Mischung von 36 g (0,12 Mol) 5,6-Dichlor-2-trifluormethyl-1H-benzimidazol, 33 g (0,24 Mol) gepulvertem Kaliumcarbonat und 300 ml Essigester tropft man bei Rückflusstemperatur eine Lösung von 14,1 g (0,15 Mol) (Chlormethyl-ethylether in 40 ml Essigester und erhitzt nach beendeter Zugabe für weitere 4 Stunden auf Siedetemperatur. Zur Aufarbeitung wird das abgekühlte Reaktionsgemisch zweimal mit jeweils 150 ml Wasser gewaschen, über Natriumsulfat getrocknet und im Vakuum eingeengt. Der Rückstand wird durch Chromatographie an Kieselgel (Laufmittel: Dichlormethan) gereinigt.

Man erhält 32,4 g (83 % der Theorie) an 5,6-Dichlor-1-ethoxymethyl-2-trifluormethyl-benzimidazol vom Schmelzpunkt 89-92°C.

In entsprechender Weise und gemäß den allgemeinen Angaben zur Herstellung erhält man die folgenden substituierten Benzimidazole der allgemeinen Formel (I):

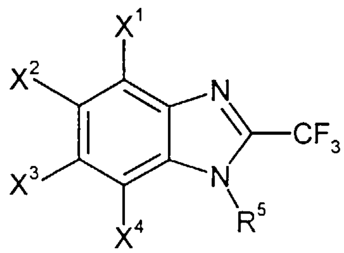

(I),

mit $R^5$ =

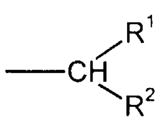

| Bsp.-Nr. | $X^1$ | $X^2$ | $X^3$ | $X^4$ | $R^1$ | $R^2$ | physikalische Eigenschaften |
|---|---|---|---|---|---|---|---|
| 2 | Br | H | $CF_3$ | H | H | 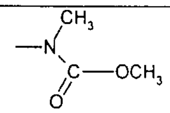 | Fp. 90-90°C |
| 3 | Br | H | $CF_3$ | H | H | 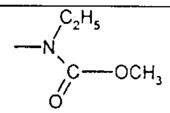 | Fp. 70-74°C |
| 4 | Br | H | $CF_3$ | H | H | 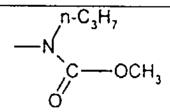 | Fp. 75-79°C |
| 5 | H | $F_2CH$-$CF_2$-O- (H) | H ($F_2CH$-$CF_2$-O-) | H | H | -O-$C_2H_5$ | |
| 6 | Br (H) | H ($CF_3$) | $CF_3$ (H) | H (Br) | H | -O-i-$C_3H_7$ | $^1$H-NMR*): 5,94; 6,00 (63:37) |
| 7 | Br (H) | H ($CF_3$) | $CF_3$ (H) | H | H | -O-n-$C_3H_7$ | Fp. 70-73°C (76:24) |
| 8 | Br | H | $CF_3$ | H | H | -O-$CH_2$-C≡CH | Fp. 71-73°C |
| 9 | B (H) | H ($CF_3$) | $CF_3$ (H) | H (Br) | H | -O-$C_2H_5$ | Fp. 82-85°C (87:13) |
| 10 | Br | H | $CF_3$ | H | H | 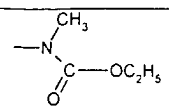 | Fp.128-130°C |

| Bsp.-Nr. | $X^1$ | $X^2$ | $X^3$ | $X^4$ | $R^1$ | $R^2$ | physikalische Eigenschaften |
|---|---|---|---|---|---|---|---|
| 11 | Br | H | $CF_3$ | H | H | CN | Fp.147-151°C |
| 12 | Br | H | $CF_3$ | H | H | 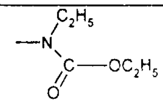 | Fp. 103-106°C |
| 13 | Br | H | $CF_3$ | H | H | 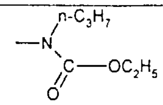 | Fp. 92-94°C |
| 14 | Br | H | $CF_3$ | H | H | 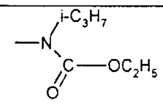 | Fp. 70-73°C |
| 15 | Br | H | $CF_3$ | H | H | 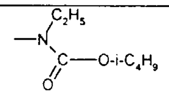 | Fp. 70-73°C |
| 16 | H | $-O-(CH_2)_3-O-$ | | H | H | $-O-C_2H_5$ | Fp. 70-74°C |
| 17 | Br | H | $CF_3$ | H | H | $-N(CH_3)_2$ | (x HCl) |
| 18 | H | $-O-(CH_2)_3-O-$ | | H | H | 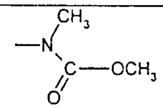 | Fp. 105-108°C |
| 19 | Br | H | $CF_3$ | H | H | 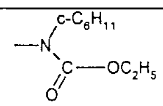 | Fp. 80-83°C |
| 20 | Br | H | $CF_3$ | H | H | 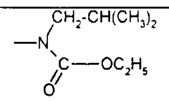 | Fp. 109-112°C |
| 21 | Br (H) | H ($CF_3$) | $CF_3$ (H) | H (Br) | H | $-S-CH_3$ | Fp. 56-60°C (1:1) |
| 22 | H | $CF_3$ (Cl) | Cl ($CF_3$) | H | H | $-COOC_2H_5$ | |
| 23 | H | $CF_3$ (Cl) | Cl ($CF_3$) | H | $-O-C_2H_5$ | $-O-C_2H_5$ | Fp. 90-92°C |
| 24 | H | $CF_3$ (Cl) | Cl ($CF_3$) | H | H | $-CO-C(CH_3)_3$ | |
| 25 | H | Cl | $CF_3$ | H | H | CN | |
| 26 | H | $CF_3$ | Cl | H | H | CN | |

| Bsp.-Nr. | $X^1$ | $X^2$ | $X^3$ | $X^4$ | $R^1$ | $R^2$ | physikalische Eigenschaften |
|---|---|---|---|---|---|---|---|
| 27 | H | $CF_3$ ($Cl$) | $Cl$ ($CF_3$) | H | H | $-OCH(CH_3)_2$ | $^1$H-NMR$^*$): A: 5.66;7.83; 8.23 B: 5.71;8.00; 8.06 |
| 28 | H | $CF_3$ ($Cl$) | $Cl$ ($CF_3$) | H | $CH_3$ | CN | $^1$H-NMR$^*$): A: 5.60; 7.80; 8.34 B: 5.65; 7.96; 8.13 |
| 29 | H | $CF_3$ ($Cl$) | $Cl$ ($CF_3$) | H | H | 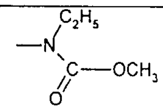 | $^1$H-NMR$^*$): A: 5.86; 7.98; 8.33 B: 5.90; 8.03; 8.21 |
| 30 | H | $CF_3$ ($Cl$) | $Cl$ ($CF_3$) | H | H | 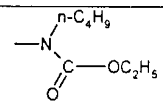 | $^1$H-NMR$^*$): A: 5.85; 7.99; 8.32 B: 5.90; 8.02; 8.22 |
| 31 | H | $CF_3$ ($Cl$) | $Cl$ ($CF_3$) | H | H | 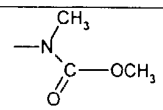 | $^1$H-NMR$^*$): A: 5.87; 7,98; 8.34 B: 5.91; 8.05; 8.22 |
| 32 | H | $-O-CF_2CF_2-O-$ | | H | H | $-O-CH(CH_3)_2$ | $^1$H-NMR$^*$): 5.61; 7.45; 7.65 |
| 33 | H | $-O-CF_2CF_2-O-$ | | H | H | CN | Fp. 132-134°C |
| 34 | H | $-O-CF_2-O-$ | | H | H | $-O-CH(CH_3)_2$ | Fp. 76-78°C |
| 35 | H | $-O-CF_2-O-$ | | H | H | CN | Fp. 145-147°C |

| Bsp.-Nr. | $X^1$ | $X^2$ | $X^3$ | $X^4$ | $R^1$ | $R^2$ | physikalische Eigenschaften |
|---|---|---|---|---|---|---|---|
| 36 | H | $CF_3$ (Br) | Br ($CF_3$) | H | H | $-O-CH(CH_3)_2$ | $^1H$-NMR*): A: 5.65; 8.03; 8.23 B: 5.69; 8.05; 8.20 |
| 37 | H | $CF_3$ (H) | H ($CF_3$) | H | H | $-O-C_2H_5$ | $^1H$-NMR*): A: 5.38; 7.18-7.94; B: 5.40 |
| 38 | H | $CF_3$ (H) | H ($CF_3$) | H | H | OH | $^1H$-NMR*): 2.2; 7.76; 8.1 |
| 39 | H | $CF_3$ (Br) | Br ($CF_3$) | H | H | $-O-C_2H_5$ | $^1H$-NMR*): A: 5.64; 8.03; 8.21 B: 5.72; 8.06; 8.18 |
| 40 | H | $CF_3$ | Br | H | H | $-O-C_2H_5$ | Fp. 66°C |
| 41 | H | Br | $CF_3$ | H | H | $-O-C_2H_5$ | $^1H$-NMR*): B: 5.72; 8.05; 8.17 |
| 42 | H | $CF_3$ (Br) | Br ($CF_3$) | H | H | $-O-n-C_3H_7$ | $^1H$-NMR*): A: 6.67; 8.08; 8.27 B: 5.69; 8.11; 8.25 |
| 43 | H | $CF_3$ (Br) | Br ($CF_3$) | H | H | $-O-CH_2-C\equiv CH$ | $^1H$-NMR*): A: 5.51; 7.89; 8.17 B: 5.71; 7.93; 8.21 |

| Bsp.-Nr. | $X^1$ | $X^2$ | $X^3$ | $X^4$ | $R^1$ | $R^2$ | physikalische Eigenschaften |
|---|---|---|---|---|---|---|---|
| 44 | H | $CF_3$ ($Cl$) | $Cl$ ($CF_3$) | H | H | $-O-C_2H_5$ | $^1H$-NMR*): A: 5.69; 7.82; 8.23 B: 5.71; 8.00; 8.03 |
| 45 | H | $CF_3$ | $Cl$ | H | H | $-O-C_2H_5$ | Fp. 73°C |
| 46 | H | $Cl$ | $CF_3$ | H | H | $-O-C_2H_5$ | $^1H$-NMR*): B: 5.71; 8.00; 8.03 |
| 47 | H | $CF_3$ ($Cl$) | $Cl$ ($CF_3$) | H | H | $-O-CH(CH_2F)_2$ | $^1H$-NMR*): A: 5.83; 7.78; 8.03 B: 5.89; 8.01; 8.26 |
| 48 | H | $CF_3$ ($Cl$) | $Cl$ ($CF_3$) | H | H | $-O-n-C_3H_7$ | $^1H$-NMR*): A. 5.70; 7.80; 8.06; B: 5.73; 7.99; 8.21 |
| 49 | H | $CF_3$ ($Cl$) | $Cl$ ($CF_3$) | H | H | $-O-CH_2-C\equiv CH$ | $^1H$-NMR*): A: 5.73; 7.81; 8.04 B: 5.77; 8.00; 8.02 |
| 50 | H | $CF_3$ ($Cl$) | $Cl$ ($CF_3$) | H | H | 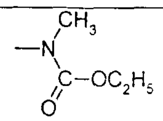 | $^1H$-NMR*): A. 5.90; 8.00; 8.21 B: 5.93; 8.03; 8.31 |
| 51 | H | $CF_3$ ($Cl$) | $Cl$ ($CF_3$) | H | H | 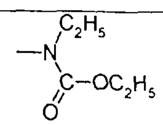 | $^1H$-NMR*): A: 5.89; 8.00; 8.21 B:5.95; 8.03; 8.33 |

| Bsp.-Nr. | $X^1$ | $X^2$ | $X^3$ | $X^4$ | $R^1$ | $R^2$ | physikalische Eigenschaften |
|---|---|---|---|---|---|---|---|
| 52 | H | $CF_3$ (Cl) | Cl ($CF_3$) | H | H | 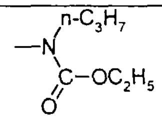 | $^1H$-NMR*): A: 5.89; 8.00; 8.22 B: 5.91; 8.04; 8.32 |
| 53 | H | -O-$CF_2$-O- | | H | H | -O-$C_2H_5$ | Fp. 92°C |
| 54 | H | -O-$CF_2$-O- | | H | H | -O-$CH(CH_2F)_2$ | Fp. 64°C |
| 55 | H | -O-$CF_2$-O- | | H | H | -O-n-$C_3H_7$ | Fp. 41°C |
| 56 | H | -O-$CF_2$-O- | | H | H | -O-$CH_2$≡CH | Fp. 87°C |
| 57 | H | -O-$CF_2$-O- | | H | H | 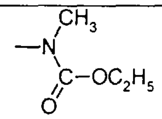 | Fp. 93°C |
| 58 | H | -O-$CF_2$-O- | | H | H | 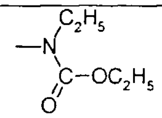 | Fp. 67°C |
| 59 | H | -O-$CF_2$-O- | | H | H | 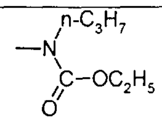 | $^1H$-NMR*): 5.89; 7.51 |
| 60 | H | -O-$CF_2$-$CF_2$-O- | | H | H | -O-$C_2H_5$ | $^1H$-NMR*) 5.63; 7.52; 7.63 |
| 61 | H | -O-$CF_2$-$CF_2$-O- | | H | H | -O-$CH(CH_2F)_2$ | $^1H$-NMR*) 5.82; 7.42; 7.68 |
| 62 | H | -O-$CF_2$-$CF_2$-O- | | H | H | 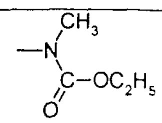 | Fp. 118°C |
| 63 | H | -O-$CF_2$-$CF_2$-O- | | H | H | 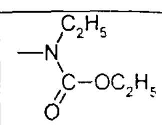 | Fp. 85°C |
| 64 | H | -O-$CF_2$-$CF_2$-O- | | H | H | 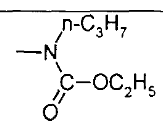 | Fp. 103°C |

| Bsp.-Nr. | $X^1$ | $X^2$ | $X^3$ | $X^4$ | $R^1$ | $R^2$ | physikalische Eigenschaften |
|---|---|---|---|---|---|---|---|
| 65 | H | $-O-CF_2-CF_2-O-$ | | H | H | $O-n-C_3H_7$ | $^1H$-NMR*): 5.75; 7.48; 7.54 |
| 66 | H | $-O-CF_2-CF_2-O-$ | | H | H | $-O-CH_2-C\equiv CH$ | $^1H$-NMR*): 5.81; 7.49; 7.68 |
| 67 | H | $-O-CF_2-CHF-O-$ ($-O-CHF-CF_2-O-$) | | H | H | $-O-C_2H_5$ | $^1H$-NMR*): 5.84; 7.64; 7.71 |
| 68 | H | $-O-CF_2-CHF-O-$ ($-O-CHF-CF_2-O-$) | | H | H | $-O-CH(CH_2F)_2$ | $^1H$-NMR*): 5.81; 6.01; 7.35; 7.61 |
| 69 | H | $-O-CF_2-CHF-O-$ ($-O-CHF-CF_2-O-$) | | H | H | $-O-n-C_3H_7$ | $^1H$-NMR*): 5.70; 6.03; 7.50; 7.60 |
| 70 | H | $-O-CF_2-CHF-O-$ ($-O-CHF-CF_2-O-$) | | H | H | $-O-CH_2-C\equiv CH$ | $^1H$-NMR*): 5.56; 6.00; 7.46; 7.54 |
| 71 | H | $-O-CF_2-CHF-O-$ ($-O-CHF-CF_2-O-$) | | H | H | 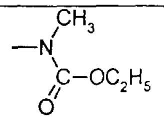 | $^1H$-NMR*): 5.78; 6.01; 7.43; 7.57 |
| 72 | H | $-O-CF_2-CHF-O-$ ($-O-CHF-CF_2-O-$) | | H | H | 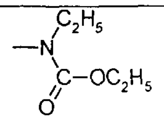 | $^1H$-NMR*): 5.80; 6.00; 7.45; 7.48 |
| 73 | H | $-O-CF_2-CHF-O-$ ($-O-CHF-CF_2-O-$) | | H | H | 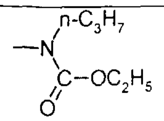 | $^1H$-NMR*): 5.85; 6.05; 7.53-7.68 |
| 74 | H | $-O-CF_2-CHF-O-$ ($-O-CHF-CF_2-O-$) | | H | H | $-CO-OC_2H_5$ | $^1H$-NMR*): 4.98; 6.03; 7.09; 7.63 |
| 75 | H | $-O-CF_2-CHF-O-$ ($-O-CHF-CF_2-O-$) | | H | $CH_3$ | $-CO-OC_2H_5$ | $^1H$-NMR*): 1.86; 6.01; 7.19; 7.62 |

| Bsp.-Nr. | $X^1$ | $X^2$ | $X^3$ | $X^4$ | $R^1$ | $R^2$ | physikalische Eigenschaften |
|---|---|---|---|---|---|---|---|
| 76 | H | $-O-CF_2-CClF-O-$ ($-O-CClF-CF_2-O-$) | | H | H | $-O-C_2H_5$ | $^1H$-NMR*): 2.35; 7.15-7.98 |
| 77 | H | $-O-CF_2-CClF-O-$ ($-O-CClF-CF_2-O-$) | | H | H | $-O-n-C_3H_7$ | |
| 78 | H | $-O-CF_2-CClF-O-$ ($-O-CClF-CF_2-O-$) | | H | H | $-O-CH_2-C{\equiv}CH$ | |
| 79 | H | 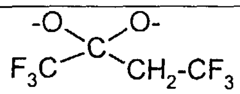 | | H | H | $-O-C_2H_5$ | $^1H$-NMR*): 5.62, 7.28; 7.32 |
| 80 | H | 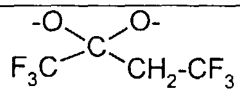 | | H | H | 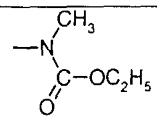 | $^1H$-NMR*): A: 5.78; 7.32; 7.44 B: 5.80; 7.32; 7.44 |
| 81 | H | 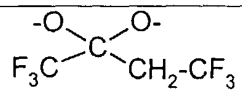 | | H | H | 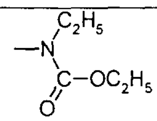 | $^1H$-NMR*): A: 5.76; 7.30; 7.42 B: 5.78; 7.30; 7.42 |
| 82 | H | 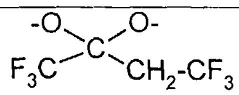 | | H | H | 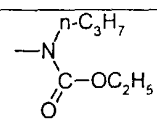 | $^1H$-NMR*): A: 5.76; 7.32; 7.42 B: 5.78; 7.32; 7.42 |
| 83 | H | $CF_3O$ (H) | H ($CF_3O$) | H | H | $-O-C_2H_5$ | |
| 84 | H | $CF_3O$ | $CF_3O$ | H | H | $-O-C_2H_5$ | $^1H$-NMR*): 5.50; 7.78; 7.82 |
| 85 | H | $CF_3O$ | $CF_3O$ | H | H | $-O-n-C_3H_7$ | $^1H$-NMR*): 5.51; 7.75; 7.79 |

| Bsp.-Nr. | $X^1$ | $X^2$ | $X^3$ | $X^4$ | $R^1$ | $R^2$ | physikalische Eigenschaften |
|---|---|---|---|---|---|---|---|
| 86 | H | $CF_3O$ | $CF_3O$ | H | H | $-O-CH_2-C\equiv C$ | $^1H$-NMR*): 5.48, 7.76; 7.80 |
| 87 | H | $CF_3O$ | $CF_3O$ | H | H | $-O-CH(CH_2F)_2$ | $^1H$-NMR*): 5.80; 7.78; 7.84 |
| 88 | H | Cl | Cl | H | H | 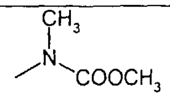 | Fp. 120-121°C |
| 89 | H | Cl | Cl | H | H | 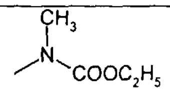 | Fp. 95-97°C |
| 90 | H | Cl | Cl | H | H | 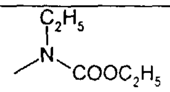 | Fp. 104-106°C |
| 91 | H | Cl | Cl | H | H | 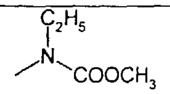 | Fp. 88-89°C |
| 92 | H | Cl | Cl | H | H | 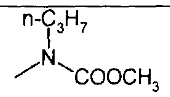 | Fp. 102-103°C |
| 93 | Cl (H) | H (Cl) | Cl (H) | H (Cl) | H | $-O-C_2H_5$ | Fp. 57-61°C (87:13) |
| 94 | Cl (H) | H (Cl) | Cl (H) | H (Cl) | H | 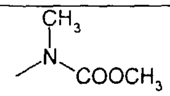 | Fp. 95-100°C (92:8) |
| 95 | H | Cl (H) | H (Cl) | H | H | $-O-CH(CH_3)_2$ | $^1H$-NMR*): 5.63; 5.65; 7.35; 7.40; 7.57; 7.78; 7.63; 7.85 |
| 96 | H | Cl (H) | H (Cl) | H | H | CN | $^1H$-NMR*): 5.15; 5.18; 7.45; 7.55; 7.83; 7.90 |

| Bsp.-Nr. | $X^1$ | $X^2$ | $X^3$ | $X^4$ | $R^1$ | $R^2$ | physikalische Eigenschaften |
|---|---|---|---|---|---|---|---|
| 97 | H | Cl<br>(H) | H<br>(Cl) | H | H | $-O-C_2H_5$ | $^1H$-NMR*): 5.43; 5.48; 7.28-8.01 |
| 98 | H | Cl | H | H | H | $-O-C_2H_5$ | Fp. 75°C |
| 99 | H | H | Cl | H | H | $-O-C_2H_5$ | Fp. 73°C |
| 100 | H | Cl<br>(H) | H<br>(Cl) | H | H | 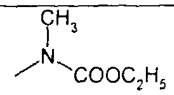 | $^1H$-NMR*): 5.88; 5.92; 7.35; 7.67; 7.92 |
| 101 | H | Cl<br>(H) | H<br>(Cl) | H | H | 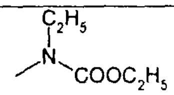 | $^1H$-NMR*): 5.88; 5.90; 7.41; 7.79; 7.81 |
| 102 | H | Cl<br>(H) | H<br>(Cl) | H | H | 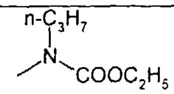 | $^1H$-NMR*): 5.89; 5.69; 7.37; 7.78; 7.78; 7.81 |
| 103 | H | Cl<br>(H) | H<br>(Cl) | H | H | OH | |
| 104 | H | F<br>(H) | H<br>(F) | H | H | $-O-C_2H_5$ | $^1H$-NMR*): A: 5.63; 7.10-7.90 B: 5.69 |
| 105 | Br | Cl | Cl | H | H | 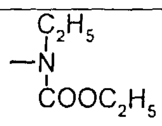 | Fp. 100-103°C |

*) Die $^1H$-NMR-Spektren wurdne in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung als δ-Wert in ppm.

Herstellung der Ausgangsverbindungen für die Verbindungen I:

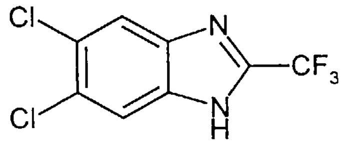

35,4 g (0,2 Mol) 4,5-Dichlorphenylen-diamin werden mit 150 ml Trifluoressigsäure für 3 Stunden auf Rückflusstemperatur erhitzt. Zur Aufarbeitung wird überschüssige Trifluoressigsäure abdestilliert und der Rückstand zwischen 100 ml Wasser und 300 ml Essigester verteilt. Die organische Phase wird abgetrennt, nacheinander mit jeweils 100 ml wässriger Natriumhydrogencarbonatlösung und Wasser gewaschen, über Natriumsulfat getrocknet und im Vakuum eingeengt. Der Rückstand wird durch Chromatographie an Kieselgel (Laufmittel: Cyclohexan/Essigester 1:1) gereinigt.

Man erhält 42,1 g (81 % der Theorie) an 5,6-Dichlor-2-trifluomiethyl-1H-benzimidazol vom Schmelzpunkt 225-230°C.

In entsprechender Weise erhält man die folgenden 1H-Benzimidazole der Formel

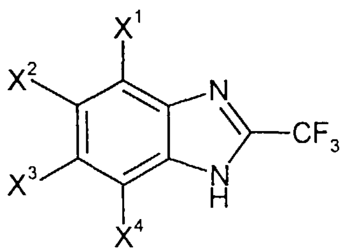

(II)

| Bsp. Nr. | $X^1$ | $X^2$ | $X^3$ | $X^4$ | physikalische Eigenschaften |
|---|---|---|---|---|---|
| II-2 | Br (H) | H ($CF_3$) | $CF_3$ (H) | H (Br) | Fp. 149-151°C |
| II-3 | H | $Cl-CH_2-SO_2-$ (H) | H ($Cl-CH_2-SO_2$) | H | Fp. 197-200°C |
| II-4 | H | $-O-CH_2-CH_2-CH_2-O-$ | | H | Fp. >230°C |
| II-5 | Br (H) | H ($Cl-CH_2-SO_2$) | $Cl-CH_2-SO_2-$ (H) | H (Br) | Fp. 180-187°C |
| II-6 | H | $CF_3$ (Br) | Br ($CF_3$) | H | Fp. 209°C |
| II-7 | H | $-O-CF_2-O-$ | | H | Fp. 242°C |
| II-8 | H | $-O-CF_2-CF_2-O-$ | | H | Fp. 235-237°C |
| II-9 | H | $-O-CF_2-CHF-O-$ ($-O-CHF-CF_2-O-$) | | H | Fp. 217°C |
| II-10 | H | -O-CFCl-CFCl-O- | | H | Fp. 185°C |
| II-11 | H | $CF_3O$ (Cl) | Cl ($CF_3O$) | H | Fp. 144°C |
| II-12 | H | 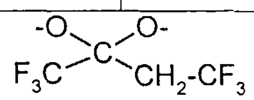 | | H | Fp. 209°C |
| II-13 | H | $CF_3O$ (H) | H ($CF_3O$) | H | Fp. 168°C |

| Bsp. Nr. | $X^1$ | $X^2$ | $X^3$ | $X^4$ | physikalische Eigenschaften |
|---|---|---|---|---|---|
| II-14 | H | $CF_3O$ | $CF_3O$ | H | Fp. 158°C |
| II-15 | H | $CF_3$ ($CH_3O$) | $CH_3O$ ($CF_3$) | H | Fp. 60°C |
| II-16 | H | $F_2CH$-$CF_2$-O- (H) | H ($F_2CH$-$CF_2$-O-) | H | Fp. 181°C |
| II-17 | H | $CF_3S$ (H) | H ($CF_3S$) | H | Fp. 174°C |
| II-18 | H | FClCH-$CF_2$-O- (H) | H (FClCH-$CF_2$-O-) | H | Fp. 57°C |
| II-19 | H | $F_3C$-$SO_2$- (H) | H ($F_3C$-$SO_2$-) | H | |
| II-20 | H | F (H) | H (F) | H | Fp. 213°C |
| II-21 | H | Cl (H) | H (Cl) | H | Fp. 193°C |
| II-22 | Cl (H) | H (Cl) | Cl (H) | H (Cl) | Fp. 165-170°C |
| II-23 | Br | Cl | Cl | H | Fp. 195-199°C |

*) Die $^1$H-NMR-Spektren wurden in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung als δ-Wert in ppm.

Chlor-(2-halogen-1-fluormethyl-ethoxy)-methane der Formel (I)

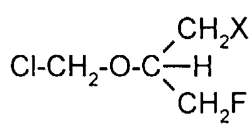

in der

X für Fluor oder Chlor steht,

(im einzelnen handelt es sich dabei um Chlor-(2-fluor-1-fluormethyl-ethoxy)-methan (Formel (I), X = Fluor) und Chlor-(2-chlor-1-fluormethyl-ethoxy)-methan (Formel (1), X = Chlor)) sind erhältlich durch Umsetzung von halogenierten Isopropanolen der Formel (II)

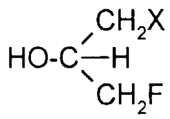

in der

X für Fluor oder Chlor steht,

bei -20 bis +20°C mit Formaldehyd und Chlorwasserstoff.

Sie dienen zur Herstellung von substituierten Benzimidazolen der Formel

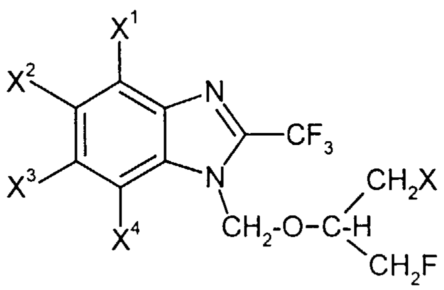

in der

X für Fluor oder Chlor stehen und

$X^1$, $X^2$, $X^3$ und $X^4$ unabhängig voneinander jeweils für Wasserstoff, Fluor, Chlor, Brom, Iod, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschieden Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, substituiertes, zweifach verknüpftes Dioxyalkylen mit 1 bis 5 Kohlenstoffatomen stehen,

wobei jedoch mindestens einer der Substituenten $X^1$, $X^2$, $X^3$ und $X^4$ verschieden von Wasserstoff und Halogen ist, aus Benzimidazolen der Formel

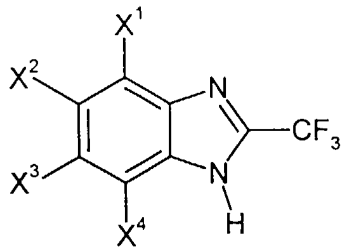

.

**Beispiel**

192 g 1,3-Difluor-2-propanol wurden mit 66 g Paraformaldehyd (fein gepulvert) versetzt. Dann wurde bei -1O°C ein kräftiger Chlorwasserstoff-Gasstrom unter Rühren eingeleitet, bis eine klare 2-phasige Mischung entstanden war. Anschließend wurde die organische Phase abgetrennt, mit Calciumchlorid getrocknet und im Vakuum fraktioniert destilliert. Mit einem Siedepunkt von 50 bis 54°C bei 20 mbar wurden 183 g (60 % der Theorie) Chlor-(2-fluor-1-fluormethyl-ethoxy)-methan erhalten. Die charakteristischen Absorptionen in den NMR-Spektren waren wie folgt:
$^{1}$H-NMR: 5,6 ppm und 4955 ppm.
$^{19}$F-NMR: -233 ppm.

Fluorierte 1,3-Benzodioxole der Formel

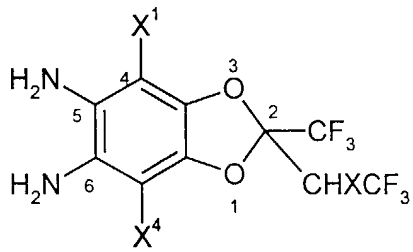

in der

X für Wasserstoff, Fluor, Chlor oder Brom stehen und

$X^1$ und $X^4$ gleich oder verschieden voneinander sein können und jeweils für Wasserstoff, Fluor, Chlor, Brom, Iod, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschieden Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, substituiertes, zweifach verknüpftes Dioxyalkylen mit 1 bis 5 Kohlenstoffatomen stehen,

sind erhältlich durch Umsetzung von 1,2-Dihydroxybenzolen der Formel

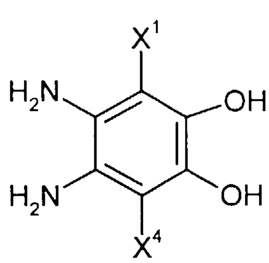

in der
$X^1$ und $X^2$ die bei Formel (I) angegebene Bedeutung haben,
in Gegenwart einer Base und eines Verdünnungsmittels bei -20 bis +200°C mit einem Hexafluorbuten der Formel (III)

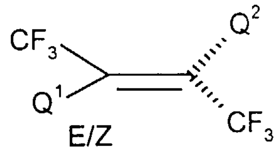

in der

$Q^1$ für Wasserstoff oder Halogen und

$Q^2$ für Halogen stehen,

oder indem man mit einer Schutzgruppe versehene 1,2-Dihydroxybenzole der Formel (IV)

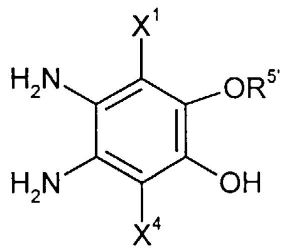

in der

$X^1$ bis $X^4$ die bei Formel (I) angegebene Bedeutung haben und

$R^{5'}$ für eine Schutzgruppe oder

$R^5$ gemeinsam mit $X^1$ für einen $-C(CH_3)_2-O-$Rest stehen,

zunächst mit einem Hexafluorbuten der Formel (III) umsetzt

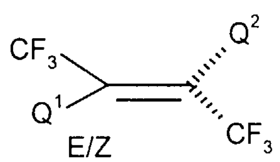

(III),

in der

$Q^1$ für Wasserstoff oder Halogen und

$Q^2$ für Halogen stehen,

so ein Zwischenprodukt der Formel (V) erhält,

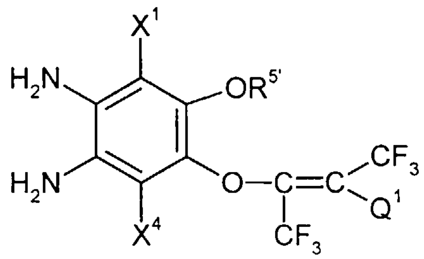

(V),

in der

$X^1$ bis $X^4$ die bei Formel (I),

$R^{5'}$ die bei Formel (IV) und

$Q^1$ die bei Formel (III)

angegebene Bedeutung haben,
aus dem Zwischenprodukt der Formel (V) die Schutzgruppe $R^{5'}$ abspaltet,
die so erhältliche OH-Verbindung mit einer Base umsetzt und so 1,3-Benzodioxole der obigen Formel erhält.

1,3-Benzodioxole die zwei benachbarte Aminogruppen enthalten können mit Trifluoressigsäure in das entsprechende Benzimidazol z.B. der folgenden Formel überführt werden

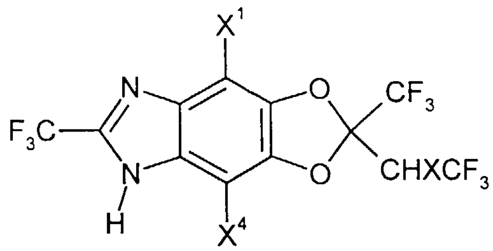

in der
$X^1$, $X^4$ und X die oben angegebene Bedeutung haben.

Aus diesen kann man durch Alkylierung Benzimidazolderivate erhalten, die im Stickstoffatom mit einem

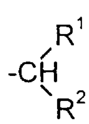

substituiert sind.

**Beispiele**

**Beispiel A**

5-Nitro-2-(2,2,2-trifluorethyl)-2-trifluormethyl-1,3-benzodioxol

Eine Lösung von 54,4 g 2-(2,2,2-Trifluorethyl)-2-trifluormethyl-1,3-benzodioxol in 75 ml Methylenchlorid wurde

bei 10°C zu einer Mischung aus 40 ml 65 gew.-%iger Salpetersäure und 40 ml konzentrierter Schwefelsäure getropft. Der Ansatz wurde 1 Stunde bei Raumtemeratur nachgerührt, dann auf Eiswasser gegossen, die organische Phase abgetrennt und die wässrige Phase mit Methylenchlorid extrahiert. Die vereinigten organischen Phasen wurden mit Wasser gewaschen, getrocknet und von leichtflüchtigen Bestandteilen befreit. Es hinterblieben 95 g des Produkts (= 86 % der Theorie) mit einem Schmelzpunkt von 87 bis 88°C.

Die NMR-Spektren zeigten folgende charakteristischen Absorptionen:
$^{19}$F-NMR: -59,0 und -69,4 ppm. $^{1}$H-NMR: 3,10 ppm.

**Beispiel B**

5,6-Dinitro-2-(2,2,2-trifluorethyl)-2-trifluormethyl-1,3-benzodioxol

317 g des Produktes aus Beispiel A wurden vorgelegt und dazu unter Rühren eine Mischung aus 250 ml 100 gew.-%iger Salpetersäure und 350 ml konzentrierter Schwefelsäure zugetropft. Die Mischung wurde 2 Stunden bei 55°C gerührt. Dann ließ man den Ansatz abkühlen und goss ihn auf Eiswasser. Das Produkt wurde mit Methylenchlorid extrahiert, mit Natriumhydrogencarbonatlösung neutral gewaschen, getrocknet und am Rotationsverdampfer von leicht flüchtigen Bestandteilen befreit. Die Ausbeute betrug 339 g (= 94 % der Theorie), der Schmelzpunkt 101 bis 103°C.

Die NMR-Spektren zeigten folgende charakteristischen Absorptionen:
$^{19}$F-NMR: -60,9 und -86,5 ppm. $^{1}$H-NMR: 3,18 ppm.

**Beispiel C**

5,6-Diamino-2-(2,2,2-trifluorethyl)-2-trifluormethyl-1,3-benzodioxol

339 g des Produkts aus Beispiel B wurden in 2000 ml Tetrahydrofuran gelöst und mit 20 g Katalysator (Palladium auf Kohle, 5 gew.-%ig) versetzt. Bei 25 bis 30 bar wurde 13 Stunden lang bei Raumtemperatur mit Wasserstoff hydriert. Anschließend wurde der Ansatz abfiltriert und das Lösungsmittel im Vakuum abgezogen. Es blieb ein Feststoff zurück. Die Ausbeute betrug 274 g (= 96 % der Theorie).
$^{19}$F-NMR: -61,2 und -86,6 ppm.
$^{1}$H-NMR: 3,02 ppm.

Fluoralkyl(en)gruppen enthaltende o-Phenylendiamine der Formel

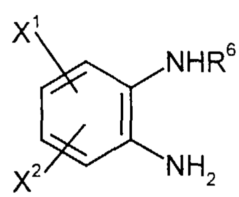

in der

$X^1$ und $X^2$ unabhängig voneinander jeweils für Wasserstoff, Fluor, Chlor, Brom, Iod, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschieden Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen, substituiertes, zweifach verknüpftes Dioxyalkylen mit 1 bis 5 Kohlenstoffatomen stehen,

wobei jedoch mindestens einer der Substituenten $X^1$ und $X^2$ verschieden von Wasserstoff und Halogen ist,
mit Ausnahme der in der EP-A 251 013 und der EP-A 487 286 beschriebenen Verbindungen, sind erhältlich indem man ein Benzolderivat der Formel

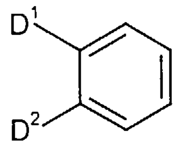

in der

$D^1$ für $CF_3O$, $CF_3S$, $CHF_2CF_2O$, CFFCl-$CF_2O$, $CF_3CHFCF_2O$, $CF_3CF_2O$, $CF_3CF_2CF_2O$, $CF_3CF_2S$ oder $CF_3$Cl-$CHFCF_2O$ und

$D^2$ für $CF_3O$, $CF_3S$, $CHF_2CF_2O$, CHFCl-$CF_2O$, $CF_3$CHF-$CF_2O$, $CF_3CF_2O$, $CF_3CF_2CF_2O$, $CF_3CHFCF_2O$, Fluor, Chlor, Brom, steht

dinitriert, die Nitrogruppen anschließend reduziert und so Verbindungen erhält, bei denen $R^1$ und $R^2$ in 4- und 5-Stellung zu den Aminogruppen stehen und die Bedeutung von $D^1$ und $D^2$ haben.

Sollen Verbindungen hergestellt werden, bei denen $X^1$ die oben angegebene Bedeutung hat und in 4-Stellung zu den Aminogruppen steht und $X^2$ für Cl oder Br in 5-Stellung zu den Aminogruppen steht, so kann man z.B. ein Nitrobenzolderivat der Formel

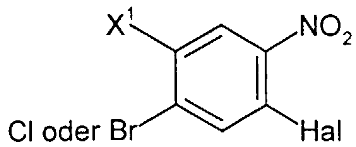

in der

$X^1$ die bei Formel (I) angegebene Bedeutung hat und

Hal für Fluor, Chlor oder Brom steht,

mit Ammoniak umsetzen, so die Hal-Gruppe gegen eine Aminogruppe austauschen und das so erhaltene Nitranilin reduzieren.

Sollen Verbindungen hergestellt werden, bei denen $X^1$ die oben angegebene Bedeutung hat und in 4-Stellung zu den Aminogruppen steht, $X^2$ für Chlor oder Brom in 6-Stellung zu den Aminogruppen steht und $R^6$ Wasserstoff bedeutet, so kann man z.B. ein Nitranilin der Formel

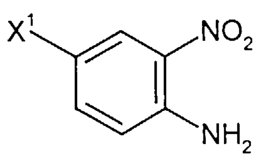

in der

$X^1$ die oben angegebene Bedeutung hat

mit einem Chlorierungs- oder Bromierungsmittel umsetzen, so ein Chlor- oder Bromatom in die meta-Stellung zur Nitrogruppe einführen und anschließend die Nitrogruppe reduzieren.

Sollen Verbindungen hergestellt werden, bei denen $X^1$ eine Donorgruppe in 4-Stellung zu den beiden Aminogruppen, $X^2$ eine Akzeptorgruppe, z.B. $CF_3$ darstellt und $R^6$ ungleich Wasserstoff ist, so kann man z.B. ein Benzolderivat der Formel

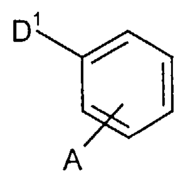

in der

$D^1$ die oben angegebene Bedeutung hat und

A für $CF_3$, $SO_2$-$C_1$-$C_6$-Alkyl, das geradkettig oder verzweigt sein kann und durch Fluor ganz oder teilweise substituiert ist,

mononitrieren (Eintritt der $NO_2$-Gruppe in para-Position zu $D^1$), die $NO_2$-Gruppe zur $NH_2$-Gruppe reduzieren, die $NH_2$-Gruppe z.B. mit Essigsäure oder Trifluoressigsäure acylieren, nochmals mononitrieren (Eintritt dieser $NO_2$-Gruppe in ortho-Position zur NHCOR-Gruppe mit R = z.B. $CH_3$ oder $CF_3$), diese $NO_2$-Gruppe zur $NH_2$-Gruppe reduzieren und gegebenenfalls, wenn man eine Verbindung der obigen mit $R^6$ = Wasserstoff herstellen will, die Acylgruppe durch Verseifung abspalten.

Die Fluoralkyl(en)gruppen enthaltenden o-Phenylendiamine, in denen $R^6$ Wasserstoff bedeutet, können zunächst mit Trifluoressigsäure zu 2-Trifluormethylbenzimidazolen der Formel

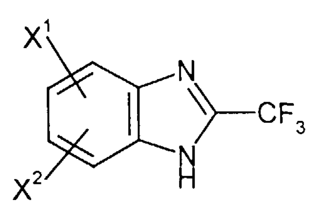

umgesetzt und dann weiter mit Verbindungen der Formel

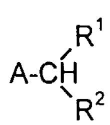

umgesetzt werden, wobei $X^1$ und $X^2$ den obigen Bedeutungsumfang annehmen,

$R^1$ und $R^2$ gleich oder verschieden für OH, CN, $NH_2$, $C_{1-6}$-Alkoxy, 1 bis 5 Halogen-$C_{1-6}$-alkoxy, $C_{1-6}$-Alkylthio, 1 bis 5 Halogen-$C_{1-6}$alkylthio, $C_{2-6}$-Alkenoxy, $C_{3-6}$-Alkinoxy, Amino oder Mono-($C_{1-6}$-alkyl)-amino, die acyliert sein können, stehen, wobei die Acylreste der aufgeführten acylierten Reste folgende Bedeutung haben: $C_1$-$C_6$-Alkoxycarbonyl, $C_1$-$C_6$-Alkylcarbonyl stehen, wobei $R^1$ auch für H stehen kann,

A eine geeignete Abgangsgruppe bedeutet.

Abgangsgruppen sind dem Fachmann bekannt und sind beispielsweise Halogen, Alkyl(Alkoxy, Aryl)sulfonyloxy, Hydroxy oder Alkoxy.

**Beispiele**

**Beispiele 1 bis 6b** (Dinitrierung und Reduktion)

**Beispiel 1b**

Zu 500 g einer Mischsäure enthalten 33 Gew.-% $HNO_3$ und 67 Gew.-% $H_2SO_4$ wurden 320 g 1,2-Bis-(2-chlor-1,1,2-trifluorethoxy)-benzol getropft. Nach einer Stunde bei 40°C wurden 250 ml 20 gew.-%iges Oleum zugetropft. Anschließend wurde auf 80°C erhitzt und 15 Stunden lang nachgerührt. Dann wurden weitere 120 ml 20 gew.-%iges Oleum und 250 g der oben angegebenen Mischsäure zugetropft. Nach 6 Stunden bei 80 bis 82°C wurde abgekühlt und auf Eis gegossen. Die organische Phase wurde abgetrennt und mit Wasser gewaschen. Nach azeotroper Trocknung mit 1,2-Dichlorethan wurden 350 g 98 Gew.-% reines 1,2-Dinitro-4,5-bis-(2-chlor-1,1,2-trifluorethoxy)-benzol erhalten (Öl, $n_{20}^{D}$: 1.4832, GC 99,1 %).

350 g dieser Dinitroverbindung wurden zu einem Gemisch aus 1,5 l Ethanol, 50 ml Wasser, 30 ml konzentrierter wässriger Salzsäure und 470 g Eisenspänen getropft und insgesamt 15 Stunden zum Sieden am Rückfluss erhitzt. Danach wurde die erkaltete Lösung abfiltriert, eingeengt und der Rückstand aus Cyclohexan umkristallisiert. Es wurden 216 g 1,2-Diamino-4,5-bis-(2-chlor-1,1,2-trifluorethoxy)-benzol mit einem Schmelzpunkt von 58 bis 60°C erhalten.

**Beispiel 2b**

Analog Beispiel 1 wurde aus 1,2-Bis-(1,1,2,3,3,3-hexafluorpropoxy)-benzol die entsprechende 4,5-Dinitroverbindung (Öl, $n_{20}^{D}$: 1,4852) und die entsprechende 4,5-Diaminoverbindung (Öl, 87 Gew.-% rein) hergestellt.

**Beispiel 3b**

Analog Beispiel 1 wurde aus 1-(1,1,2-Trifluor-2-chlorethoxy)-2-chlorbenzol die entsprechende 4,5-Dinitroverbindung (Schmelzpunkt 56 bis 57°C) und die entsprechende 4,5-Diaminoverbindung (Schmelzpunkt 67 bis 68°C) hergestellt.

**Beispiel 4b**

Analog Beispiel 1 wurde aus 1-Trifluormethoxy-2-brombenzol die entsprechende 4,5-Dinitroverbindung (Schmelzpunkt 73 bis 75°C) und die entsprechende 4,5-Diaminoverbindung (Öl, 98 Gew.-% rein, $n_{20}^{D}$: 1,5485) hergestellt.

**Beispiel 5b**

Analog Beispiel 1 wurde aus 1-Trifluormethoxy-2-chlorbenzol die entsprechende 4,5-Dinitroverbindung (Schmelzpunkt 55 bis 56°C) und die entsprechende 4,5-Diaminoverbindung (Schmelzpunkt 56 bis 57°C) hergestellt.

**Beispiel 6b**

Aus 1-(1,1,2,3,3,3-Hexafluorpropoxy)-2-chlor-benzol wurde die entsprechende 4,5-Dinitroverbindung (Öl) und die entsprechende 4,5-Diaminoverbindung (Öl) hergestellt.

**Beispiele 7 bis 12b**

Verdrückung mit Ammoniak und Reduktion

**Beispiel 7b**

In einem Autoklaven wurden 260g 3-Nitro-2,5-dichlorbenzotrifluorid, 130 ml Wasser und 10 g Tetraethylammoniumchlorid vorgelegt und 120 ml flüssiges Ammoniak aufgedrückt. Anschließend wurde auf 130°C erhitzt und für 10 Stunden bei dieser Temperatur gerührt. Nach dem Abkühlen wurde der Ansatz abfiltriert, der abgetrennte Niederschlag mit Wasser gewaschen und getrocknet. Es fielen 194 g 2-Amino-3-nitro-5-chlor-benzotrifluorid mit einem Schmelzpunkt von 67°C an.

134 g des wie oben geschrieben erhaltenen Nitranilins wurden in 800 ml Ethanol gelöst, dan 20 ml Wasser, 10 ml konzentrierte wässrige Salzsäure und 160 g Eisenspäne zugegeben. Die Mischung wurde für 15 Stunden zum Sieden am Rückfluss erhitzt, dann abgekühlt, abgesaugt, der Filterrückstand mit Dichlormethan gewaschen und anschließend die organischen Phasen unter reduziertem Druck vom Lösungsmittel befreit. Es fielen 171 g 5-Chlor-3-trifluormethyl-1,2-diaminobenzol mit einem Schmelzpunkt von 53°C an.

**Beispiel 8b**

Analog Beispiel 7 wurde aus 3-Nitro-4,6-dichlor-difluorchlormethoxybenzol zunächst 3-Nitro-4-amino-6-chlordifluorchlormethoxybenzol (Schmelzpunkt 73°C) und daraus 3,4-Diamino-6-chlor-difluorchlormethoxybenzol (Öl) erhalten.

**Beispiel 9b**

Analog Beispiel 7 wurde aus 3-Brom-5-nitro-6-chlorbenzotrifluorid zunächst 3-Brom-5-nitro-6-amino-benzotrifluorid (Schmelzpunkt 80 bis 82°C) und daraus 3-Brom-5,6-diamino-benzotrifluorid (Schmelzpunkt 52 bis 54°C) hergestellt.

**Beispiel 11b**

Analog Beispiel 7 wurde aus 3,6-Dichlor-5-nitro-benzotrifluorid zunächst 3-Chlor-5-nitro-6-amino-benzotrifluorid (Schmelzpunkt 53 bis 54°C) und daraus 3-Chlor-5,6-diamino-benzotrifluorid hergestellt.

**Beispiel 12b**

Aus 2-Brom-4-fluor-5-nitro-(1,1,2-trifluor-2-chlor)-ethoxybenzol wurde zunächst 2-Brom-4-amino-5-nitro-(1,1,2-trifluor-2-chlor-ethoxy)-benzol (Schmelzpunkt 90°C) und daraus 2-Brom-4,5-diamino-(1,1,2-trifluor-2-chlor)-ethoxybenzol hergestellt.

**Beispiel 13b**

(Halogenierung eines Nitranilins und Reduktion)

24 g fein gepulvertes 2-Nitro-4-trifluormethylmercaptoanilin wurden in 50 ml Trifluoressigsäure gelöst und bei 20°C 18 g Brom zudosiert. Dann wurde für 3 Stunden bei 20°C und für weitere 30 Minuten bei 40°C nachgerührt, die Mischung auf Wasser gegeben und das Produkt in Dichlormethan aufgenommen. Es fielen nach Entfernung des Lösungsmittels 31 g 6-Brom-2-nitro-4-trifluormethylmercapto-anilin an.

155 g des so hergestellten Nitranilins wurden in 700 ml Ethanol mit 15 ml Wasser, 10 ml konzentrierter wässriger Salzsäure und 70 g Eisenspänen für 15 Stunden zum Sieden am Rückfluss erhitzt, dann das Gemisch abfiltriert, das Filtrat unter reduziertem Druck vom Lösungsmittel befreit und das feste Rohprodukt aus Cyclohexan umkristallisiert. Es wurden 112 g 6-Brom-4-trifluormethylmercapto-1,2-diaminobenzol mit einem Schmelzpunkt von 60 bis 61°C erhalten.

**Beispiel 14b**

Analog Beispiel 13 wurden 27 g 2-Nitro-4-trifluormethylsulfonylanilin in 100ml Essigsäure mit 18 g Brom bromiert.

Nach Aufarbeitung fielen 32 g 2-Nitro-6-brom-4-trifluormethylsulfonyl-anilin an, Schmelzpunkt 147°C.

32 g des so hergestellten Nitramins wurde mit Eisenspänen in Alkohol und wässriger Chlorwasserstoffsäure reduziert. Es fielen 24 g 3-Brom-5-trifluormethylsulfonylphenylen-1,2-diamin an, Schmelzpunkt 155 bis 157°C.

**Beispiel 15b**

Analog Beispiel 14 wurden 27 g 2-Nitro-4-trifluormethylsulfonyl-anilin in 100 ml Essigsäure mit 10 g Chlor chloriert. Es fielen 29 g 2-Nitro-4-trifluormethylsulfonyl-6-chlor-anilin an, Schmelzpunkt: 138 bis 139°C.

Durch Reduktionwurden 13 g 3-Chlor-5-trifluormethylsulfonyl-1,2-phenylendiamin (Schmelzpunkt: 143 bis

145°C) erhalten.

**Beispiel 16 bis 20b**

(Nitrierung und Reduktion in 2 Stufen)

**Beispiel 16b**

263 g 4-(2,6-Dichlor-4-trifluormethyl)-phenoxy-acetanilid wurden in 1100 ml Dichlormethan gelöst und bei 10°C vorgelegt. Dann wurden bei dieser Temperatur 88 g 98 gew.-%ige Salpetersäure zugetropft. Es wurde 1 Stunde bei 10°C und 2 weitere Stunden bei 30°C nachgerührt. Nach der Zugabe von 300 ml Wasser wurden die Phasen getrennt und die organische Phase unter reduziertem Druck von Dichlormethan befreit. Es verblieben 253 g 2-Nitro-4-(2,6-dichlor-4-trifluormethyl-phenoxy)-acetanilid mit einem Schmelzpunkt von 138 bis 140°C.

91 g des so hergestellten Acetanilids wurden in 800 ml Dioxan gelöst, 10 g Raney-Nickel zugegeben und bei 25 bis 45°C in einer Hydrierapparatur mit maximal 50 bar Wasserstoffdruck hydriert. Nach Entspannen und Filtration wurde das Dioxan bei leichtem Vakuum abdestilliert. Es verblieben 65 g 2-Amino-4-(2,6-dichlor-4-trifluormethyl-phenoxy)-acetanilid mit einem Schmelzpunkt von 222 bis 223°C.

**Beispiel 17b**

Analog zu Beispiel 16 wurde aus 3-Trifluormethyl-4-methoxy-acetanilid zunächst 3-Trifluormethyl-4-methoxy-6-nitro-acetanilid (Schmelzpunkt 143 bis 144°C) und daraus 3-Trifluormethyl-4-methoxy-6-amino-acetanilid (Schmelzpunkt 164 bis 165°C) hergestellt.

**Beispiel 18b**

Analog Beispiel 16 wurde aus 3-Trifluormethyl-4-fluor-trifluormethylacetanilid zunächst 3-Trifluormethyl-4-fluor-6-nitro-trifluormethylacetanilid (Schmelzpunkt 78°C) und daraus 3-Trifluormethyl-4-fluor-6-amino-trifluormethylacetanilid (Schmelzpunkt 92 bis 93°C) hergestellt.

**Beispiel 19b**

Analog Beispiel 16 wurde aus 3-Trifluormethyl-4-brom-trifluormethylacetanilid zunächst 3-Trifluormethyl-4-brom-6-nitro-trifluormcthylacetanilid (Schmelzpunkt 110 bis 112°C) und daraus 3-Trifluormethyl-4-brom-6-amino-trifluormethylacetanilid (Schmelzpunkt 63 bis 65°C) hergestellt.

**Beispiel 20b**

Analog Beispiel 16 wurde aus 3-Trifluormethylthio-4-chlor-trifluormethylacetanilid zunächst 3-Trifluormethylthio-4-chlor-6-nitro-trifluormethylacetanilid (Schmelzpunkt 99 bis 100°C) und daraus 3-Trifluormethylthio-4-chlor-6-amino-trifluormethylacetanilid (Schmelzpunkt: 88 bis 90°C) hergestellt.

**Beispiel 21b**

0,2 Mol 3-Brom-5-trifluormethyl-phenylen-diamin wurden mit 150 ml Trifluoressigsäure für 3 Stunden auf Rückflusstemperatur erhitzt. Zur Aufarbeitung wurde überschüssige Trifluoressigsäure abdestilliert und der Rückstand zwischen 100 ml Wasser und 300 ml Essigester verteilt. Die organische Phase wurde abgetrennt, nacheinander mit jeweils 100 ml wässriger Natriumhydrogencarbonatlösung und Wasser gewaschen, über Natriumsulfat getrocknet und im Vakuum eingeengt. Der Rückstand wurde durch Chromatographie an Kieselgel (Laufmittel: Cyclohexan/Essigester 1:1) gereinigt.

Man erhielt 4-Brom-6-trifluormethyl-2-trifluormethyl-1H-benzimidazol vom Schmelzpunkt 149 bis 151 °C.

**Beispiel 22b**

0,03 Mol 4-Brom-6-trifluormethyl-2-trifluormethyl-1H-benzimidazol und 0,06 Mol pulverisiertes Kaliumcarbonat wurden in 70 ml Essigester für 15 Minuten auf Rückflusstemperatur erhitzt, anschließend mit 3,9 g (0,04 Mol) Chlormethyl-methylthioether in 20 ml Essigester versetzt und unter Rühren für weitere 4 Stunden auf Rückflusstemperatur erhitzt. Zur Aufarbeitung wurde die abgekühlte Reaktionsmischung zweimal mit jeweils 40 ml Wasser gewaschen, über Natriumsulfat getrocknet, im Vakuum eingeengt und der Rückstand durch Chromatographie an Kieselgel (Laufmittel: Dichlormethan) gereinigt.

Man erhielt 1-Methylthiomethyl-4-brom-6-trifluormethyl-2-trifluormethyl-benzimidazol vom Schmelzpunkt 56 bis 60°C.

**Patentansprüche**

**1.** Verwendung von substituierten Benzimidazolen der Formel (I)

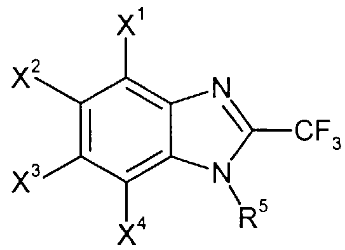

(I),

in welcher

$X^1$, $X^2$, $X^3$ und $X^4$ unabhängig voneinander jeweils für Wasserstoff, Fluor, Chlor, Brom, Iod, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschieden Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 5 Kohlenstoffatomen stehen,

wobei jedoch mindestens einer der Substituenten $X^1$, $X^2$, $X^3$ und $X^4$ verschieden von Wasserstoff und Halogen ist,

$R^5$ für $C_{1-4}$-Alkyl steht, das ein- oder mehrfach, gleich oder verschieden substituiert ist durch OH, CN, $NH_2$, $C_{1-6}$-Alkoxy, 1 bis 5 Halogen-$C_{1-6}$-Alkoxy, $C_{1-6}$-Alkylthio, 1 bis 5 Halogen-$C_{1-6}$-alkylthio, $C_{2-6}$-Alkenoxy, $C_{3-6}$-Alkinoxy, Amino oder Mono-($C_{1-6}$-alkyl)-amino, die acyliert sein können,

wobei die Acylreste der aufgeführten acylierten Reste folgende Bedeutung haben: $C_{1-6}$-Alkoxycarbonyl, $C_{1-6}$-Alkylcarbonyl
zur Herstellung von Mitteln zur Bekämpfung parasitärer Protozoen, insbesondere Coccidien.

**2.** Verwendung gemäß Anspruch 1 von substituierten Benzimidazolen der Formel (I), in welcher

$X_1$ und $X_4$ für gleiche oder verschiedene Reste aus der Gruppe H, $C_{1-4}$-Alkyl, das durch Fluor oder Chlor perhalogeniert ist, Halogen, stehen,

einer der Reste $X^2$ oder $X^3$ für halogen-substituierte Reste der Gruppe $C_{1-4}$-Alkyl, Alkylthio steht und der andere Rest $X^2$ oder $X^3$ für Wasserstoff oder Halogen steht;
die Reste $X^2$ und $X^3$ können gemeinsam mit den angrenzenden C-Atomen für einen Dioxolanyl- oder Dioxanylring stehen, die durch Halogen, Trifluormethyl oder Trifluorethyl substituiert sein können;

$R^5$ für Methyl steht, das substituiert ist durch OH, CN, $C_{1-4}$-Halogenalkoxy, Amino oder Mono-($C_{1-4}$-alkyl)amino, die acyliert sein können durch $C_{1-4}$-Alkoxycarbonyl.

**3.** Verwendung gemäß Anspruch 1 von Verbindungen der Formel (I), in welcher die Reste folgende Bedeutung haben:

$X^1$ und $X^4$ stehen für gleiche oder verschiedene Reste, Wasserstoff, Chlor, Brom, Trifluormethyl;

einer der Reste $X^2$ oder $X^3$ steht für Trifluormethyl,
der andere Rest $X^2$ oder $X^3$ steht für Wasserstoff oder Halogen,

$R^5$ hat die in den Ansprüchen 1 und 2 angegebene Bedeutung.

## Claims

**1.** Use of substituted benzimidazoles of the formula (I)

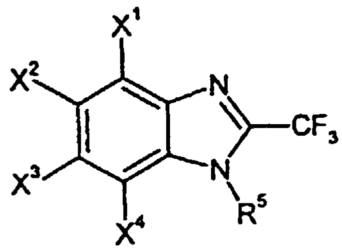

(I),

in which

$X^1$, $X^2$, $X^3$ and $X^4$ independently of one another in each case represent hydrogen, fluorine, chlorine, bromine, iodine, in each case straight-chain or branched halogenoalkyl, halogenoalkoxy, halogenoalkylthio, halogenoalkylsulphinyl, halogenoalkylsulphonyl having in each case 1 to 6 carbon atoms and 1 to 13 identical or different halogen atoms, or divalent dioxyalkylene having 1 to 5 carbon atoms which is optionally monosubstituted or polysubstituted by identical or different substituents consisting of halogen and/or straight-chain or branched alkyl having 1 to 4 carbon atoms and/or straight-chain or branched halogenoalkyl having 1 to 4 carbon atoms and 1 to 9 identical or different halogen atoms,

but where at least one of the substituents $X^1$, $X^2$, $X^3$ and $X^4$ is other than hydrogen and halogen,

$R^5$ represents $C_{1-4}$-alkyl which is monosubstituted or polysubstituted by identical or different substituents consisting of OH, CN, $NH_2$, $C_{1-6}$-alkoxy, 1 to 5 halogeno-$C_{1-6}$-alkoxy, $C_{1-6}$-alkylthio, 1 to 5 halogeno-$C_{1-6}$-alkylthio, $C_{2-6}$-alkenoxy, $C_{3-6}$-alkinoxy, amino or mono-($C_{1-6}$-alkyl)-amino, it being possible for these to be acylated,

the acyl radicals of the acylated radicals cited having the following meaning: $C_{1-6}$-alkoxycarbonyl, $C_{1-6}$-alkylcarbonyl,
for the preparation of products for controlling parasitic protozoa, in particular coccidia.

**2.** Use according to Claim 1 of substituted benzimidazoles of the formula (I) in which

$X_1$ and $X_4$ represent identical or different radicals from the group consisting of H, $C_{1-4}$-alkyl which is perhalogenated by fluorine or chlorine, halogen,

one of the radicals $X^2$ or $X^3$ represents halogen-substituted radicals from the group consisting of $C_{1-4}$-alkyl, alkylthio, and the other radical $X^2$ or $X^3$ represents hydrogen or halogen;
it being possible for the radicals $X^2$ and $X^3$ together with the adjacent C atoms to represent a dioxolanyl or dioxanyl ring, it being possible for these to be substituted by halogen, trifluoromethyl or trifluoroethyl;

$R^5$ represents methyl which is substituted by OH, CN, $C_{1-4}$-halogenoalkoxy, amino or mono-($C_{1-4}$-alkyl)amino, it being possible for these to be acylated by $C_{1-4}$-alkoxycarbonyl.

**3.** Use according to Claim 1 of compounds of the formula (I) in which the radicals are defined as follows:

$X^1$ and $X^2$ represent identical or different radicals, hydrogen, chlorine, bromine, trifluoromethyl;

one of the radicals $X^2$ or $X^3$ represents trifluoromethyl,
the other radical $X^2$ or $X^3$ represents hydrogen or halogen,

$R^5$ is as defined in Claims 1 and 2.

## Revendications

**1.** Utilisation de benzimidazoles substitués de la formule (I) :

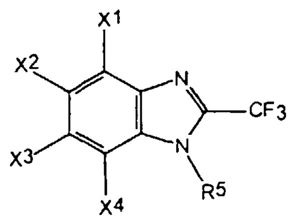

(I)

dans laquelle :

$X^1$, $X^2$, $X^3$ et $X^4$ représentent indépendamment l'un de l'autre, chaque fois un atome d'hydrogène, de fluor, de chlore, de brome, d'iode, un groupement halogénoalcoyle, halogénoalcoxy, halogénoalcoylthio, halogénoalcoylsulfinyle, halogénoalcoyisulfonyle linéaires ou ramifiés, ayant chaque fois 1 à 6 atomes de carbone et 1 à 13 atomes d'halogène identiques ou différents, ou un groupement dioxyalcoylène relié deux fois, ayant 1 à 5 atomes de carbone, facultativement substitué une ou plusieurs fois, de manière identique ou différente par un atome d'halogène et/ou un groupement alcoyle linéaire ou ramifié ayant 1 à 4 atomes de carbone et/ou halogénoalcoyle linéaire ou ramifié ayant 1 à 4 atomes de carbone et 1 à 9 atomes d'halogène identiques ou différents,

où cependant, au moins l'un des substituants $X^1$, $X^2$, $X^3$ et $X^4$ est différent de l'hydrogène et des halogènes,

$R^5$ représente un groupement alcoyle en $C_{1-4}$, qui est substitué une ou plusieurs fois, de manière identique ou différente, par OH, CN, $NH_2$, alcoxy en $C_{1-6}$, alcoxy en $C_{1-6}$ 1 à 5 fois halogéné, alcoylthio en $C_{1-6}$, alcoylthio en $C_{1-6}$ 1 à 5 fois halogéné, alcénoxy en $C_{2-6}$, alcynoxy en $C_{3-6}$, amino ou mono(alcoyl en $C_{1-6}$)amino, qui peuvent être acylés,

où le radical acyle des restes acylés indiqués a la signification suivante : (alcoxy en $C_{1-6}$)carbonyle, (alcoyl en $C_{1-6}$)carbonyle, pour préparer des agents pour combattre les protozoaires parasites, en particulier les coccidies.

**2.** Utilisation suivant la revendication 1, de benzimidazoles substitués de formule (I), dans laquelle

$X^1$ et $X^4$ représentent des restes identiques ou différents parmi le groupe constitué de H, alcoyle en $C_{1-4}$, qui est perhalogéné par le fluor ou le chlore, halogène,

l'un des reste $X^2$ ou $X^3$ représente un reste halogéno-substitué du groupe constitué des radicaux alcoyle en $C_{1-4}$, alcoylthio et l'autre reste $X^2$ ou $X^3$ représente un atome d'hydrogène ou d'halogène ;
les restes $X^2$ et $X^3$ peuvent également représenter ensemble, les atomes C voisins d'un cycle dioxolannyle ou dioxannyle, qui peuvent être substitués par un atome d'halogène, un groupement trifluorométhyle ou trifluoroéthyle ;

$R^5$ représente un groupement méthyle, qui est substitué par OH, CN, halogénoalcoxy en $C_{1-4}$, amino ou mono(alcoyle en $C_{1-4}$)amine, qui peuvent être acylés par (alcoxy en $C_{1-4}$)carbonyle.

**3.** Utilisation suivant la revendication 1, de composés de formule (I), dans laquelle les restes ont la signification suivante :

$X^1$ et $X^4$ représentent des restes identiques ou différents parmi l'hydrogène, le chlore, le brome et le groupement trifluorométhyle ;

l'un des restes $X^2$ ou $X^3$ représente le groupement trifluorométhyle,
l'autre des restes $X^2$ ou $X^3$ représente un atome d'hydrogène ou d'halogène,

$R^5$ a les significations données aux revendications 1 et 2.